Europäisches Patentamt

European Patent Office

Office européen des brevets

(11) Publication number: **0 363 102 B1**

(12) **EUROPEAN PATENT SPECIFICATION**

(45) Date of publication of patent specification: **25.05.94**

(21) Application number: **89309992.9**

(22) Date of filing: **29.09.89**

(51) Int. Cl.5: **B65D 65/38**, B65F 1/00, A23L 3/00, A61B 19/02, B29C 47/00, //B29K67:00

(54) Polybutylene terephtalate resin bag.

(30) Priority: **05.10.88 JP 251672/88**

(43) Date of publication of application:
**11.04.90 Bulletin 90/15**

(45) Publication of the grant of the patent:
**25.05.94 Bulletin 94/21**

(84) Designated Contracting States:
**AT BE CH DE ES FR GB GR IT LI LU NL SE**

(56) References cited:
EP-A- 0 287 246      EP-A- 0 299 643
EP-A- 0 323 128      DE-A- 3 317 300
FR-A- 2 242 220      GB-A- 989 080
GB-A- 1 026 133      GB-A- 2 141 723
US-A- 4 243 712

(73) Proprietor: **POLYPLASTICS CO. LTD.**
**3-13, Azuchicho, 2-chome**
**Chuo-Ku Osaka-shi Osaka 541(JP)**

(72) Inventor: **Nedzu, Shigeru**
**324 Miyashita**
**Fuji-shi Shizuoka(JP)**
Inventor: **Fukasawa, Jun**
**324 Miyashita**
**Fuji-shi Shizuoka(JP)**

(74) Representative: **Jackson, Peter et al**
**HYDE, HEIDE & O'DONNELL**
**10-12 Priests Bridge**
**London SW15 5JE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a bag for which heat sterilization is required. More particularly, the present invention relates to a bag for disposal of waste, which is suitable for a high-temperature sterilization treatment with hot air, steam or hot water when waste is contained therein.

(Prior Art)

Polyethylene bags have been utilized for containing waste, for preserving food and the like. However, where heat sterilization is necessary for waste discharged from a hospital or the like (blood, body fluids, excreta, filth or the like), or for food, the packed bag as a whole is heated a temperature of up to about 130°C, which results in breaking or splitting of a polyethylene bag because of its insufficient heat resistance. For avoiding this disadvantage, use of a bag formed of polypropylene has been tried but this has a large gas permeability though it has a heat resistance, and therefore, if used for containing waste, permits the escape of unpleasant smells generated during the sterilization and makes the bag unpleasant to handle. As the means for solving this problem, there is used a two-layer bag comprising an inner bag composed or polyethylene film containing an adsorbent incorporated thereinto and an outer bag composed of a polypropylene film. However, this bag is so expensive that it is not preferred from the economic viewpoint.

As a film having excellent heat resistance, gas barrier properties and a flavor-retaining properties, there can be mentioned a polyethylene terephthalate resin film, but since this film is biaxially oriented and cannot be heat-sealed, the film cannot be used for a single-layer bag.

(Summary of the Invention)

We have made research with a view to developing a bag suitable for containing a material for which heat sterilization is necessary, for example, odoriferous waste generated in a hospital or the like, and having a sufficient heat resistance even at heat sterilization with hot air, steam or hot water, a strength sufficient to hold contents even by a single-layer bag, and good gas-barrier properties so as not to release odours generated by waste sterilization, and so as to provide an excellent flavor-retaining property. As a result, we have found that a film composed of a specific polybutylene terephthalate resin is suitable for attaining these objects. We have now completed the present invention based on this finding.

More specifically, in accordance with the present invention, there is provided a polybutylene terephthalate bag, which has a thickness of 10 to 200 $\mu$m and an oxygen permeation coefficient of 5.0 x $10^{-10}$ cc.cm/(cm$^2$.sec.cmHg) or below at 100°C. This bag does not emit a smell to the outside and is suitable for a heat treatment of contents. Food, waste or the like, of which heat sterilization is required, is placed in the bag, which is then closed according to an appropriate method and the closed bag is treated with hot air, steam or hot water.

In the present invention, the term "polybutylene terephthalate" refers to a polyester in which main recurring units are composed of butylene terephthalate. More specifically, the polybutylene terephthalate is a polyester formed by condensing 1,4-butane-diol with terephthalic acid or a lower alcohol ester thereof. A copolymer compound mainly of polybutylene terephthalate can also be used.

Known additives customarily used for thermoplastic resins and thermosetting resins, for example, plasticizers, stabilizers such as antioxidants and ultraviolet ray absorbers, antistatic agents, surface active agents, flame retardants, flame retarding assistants, colorants such as dyes and pigments, and flowability improvers such as lubricants and crystallization promoters (nucleating agents), can be used according to the required properties, so far as attainment of the object of the present invention is not hindered. Moreover, a small amount of other thermoplastic resin or an inorganic filler can be auxiliarily used according to need, so far as attainment of the intended effect of the present invention is not hindered.

A film of the polybutylene terephthalate resin of the present invention is prepared by the inflation molding method or T-die method using the polybutylene terephthalate resin. The film obtained by the inflation molding is advantageous in various points over the film obtained by the T-die method. For example, the tensile strength is high, the gas barrier property is excellent, and the film can be manufactured at a low cost. Therefore, it is preferred that the film prepared by the inflation molding method be used.

The inflation method is often used for formation of a polyethylene film, and according to this molding method, a plastic material is extruded in the form of a tube from an annular extrusion molding orifice and a fluid such as air is blown into the tube to inflate the tube and form a tubular film. According to the T-die method, a plastic material is extruded in the form of a film from a linear slit-shaped extrusion molding

2

orifice. The inflation method is overwhelmingly excellent in the productivity. However, resin materials that can be applied to the inflation method are limited, and not all of resins can be molded according to the inflation method. Thus it has been considered that a polybutylene terephthalate resin cannot be treated by the inflation method. However, it has been found that a polybutylene terephthalate having an intrinsic viscosity of at least 1.0, preferably 1.0 to 2.5, can be formed into a film and that this film has properties suitable for attaining the object of the present invention, as compared with plastic films heretofore commercially manufactured.

When a polybutylene terephthalate resin having an intrinsic viscosity lower than 1.0 is used, formation of a film is difficult, however varied the inflation conditions may be. In the case where a polybutylene terephthalate resin having an intrinsic viscosity lower than 1.0 has to be used because of required properties or the like, a film can be prepared according to the T-die method. Even in the T-die method, the intrinsic viscosity of the polybutylene terephthalate resin used is preferably at least 0.5 and especially preferably at least 0.7.

According to the inflation molding method, a cylindrical (tubular) film is obtained, and a bag can be manufactured at a low cost very easily by cutting the cylindrical film in a predetermined length and heat-sealing one end. Furthermore, since the film obtained according to the inflation method without quenching can be made opaque, the contents cannot be seen from the outside and the bag is preferably used for containing waste. In addition, this bag is advantageous in that the tensile strength is high and the bag has excellent gas barrier property and flavor-retaining property.

The film used in the present invention, especially the inflation film, has a heat resistance and when waste or the like is filled as the contents, a sufficient strength required of the bag is retained, and the film has good gas barrier property, flavor-retaining property and heat sealability. Accordingly, the film is suitable as the material of the bag of this type.

Namely, the bag of the present invention is characterized in that the bag has an excellent gas barrier property and a high mechanical strength, and these excellent properties are not substantially degraded even at a steam or hot water treatment temperature. More specifically, the thickness is 10 to 200 $\mu$m, and the oxygen permeation coefficient at 100°C is 5.0 x $10^{-10}$ cc•cm/(cm$^2$•sec•cmHg) or below as determined according to the method of ASTM D-1434. It is preferred that the thickness be 20 to 100 $\mu$m and the oxygen permeation coefficient be 3.0 x $10^{-10}$ cc•cm/(cm$^2$•sec•cmHg) or below. It is indispensable that when the bag is cooled after the hot air, steam or hot water treatment, the reduction ratio of the tensile strength and elongation under conditions of a temperature of 23°C and a relative humidity of 50% should be 20% or less preferably 10% or less. If the oxygen permetation coefficient exceeds the above-mentioned level, emission of a smell at the treatment becomes conspicuous, and if the reduction ratio of the tensile strength and elongation exceeds 20%, the risk of breaking of the bag increases.

The oxygen permeation coefficient of a polypropylene film measured under the same high-temperature conditions is 1.1 x $10^{-8}$ cc•cm/(cm$^2$•sec•cmHg), so that the polypropylene film is much inferior to the polybutylene terephthalate film and the permeation of a bad smell or the like is conspicuous in the polypropylene film.

In the bag of the present invention, the thickness is 10 to 200 $\mu$m, preferably 20 to 100 $\mu$m. If the thickness is smaller than 10 $\mu$m, the mechanical strength characteristics (tensile strength, elongation and tear strength) required of a bag of this type at the above-mentioned high temperature are not manifested. If the thickness is larger than 200 $\mu$m, sealing of the end of the opening becomes difficult and the softness is insufficient. The polybutylene terephthalate film has a strength several times as high as those of other thermoplastic resin films (such as polyethylene and polypropylene films), when compared based on the same thickness. Accordingly, it is possible to reduce the thickness of the bag, and the polybutylene terephthalate bag is advantageous from the economic viewpoint.

The bag of the invention is preferred to have a humidity permeation of 7 x $10^{-3}$ g.m/m$^2$.day.

The bag of the invention is used as a container for bad smelling wastes which should be sterilized with heated air, heated water or water vapor from outside. In the use, a period of time for sterilization can be reduced because of a high humid permeation of the bag. In the invention, the sterilization can be effected for 15 to 40 minutes by treatment with saturated water vapor at 120 to 128 degree C.

The temperature dependency of the tensile strength of the polybutylene terephthalate resin film is smaller than that of the polypropylene resin film, and after the heat sterilization, it is possible to take out the bag still in the hot state so that the sterilization treatment step can be simplified. The tensile strength of the polybutylene terephthalate resin film is about three times as high as that of the polypropylene film at 60°C, and the tensile strength of the polybutylene terephthalate resin film at 60°C is higher than the tensile strength of the polypropylene film at normal temperature (23°C).

(Examples)

The present invention will now be described in detail with reference to the following Examples that by no means limit the scope of the invention.

Example 1 and Comparative Examples 1 and 2

A polybutylene terephthalate (PBT) inflation film bag having a thickness of 70 $\mu$m, a length of 1 m, a width of 50 cm, an intrinsic viscosity of 1.4 measured by using an o-chlorophenol solution at 25°C and an oxygen permeation coefficient of 2.0 x $10^{-10}$ cc•cm/(cm$^2$•sec•cmHg), a polypropylene (PP) inflation film bag having the same size as mentioned above and an oxygen permeation coefficient of 1.1 x $10^{-8}$ cc•cm/-(cm$^2$•sec•cmHg) and a polyethylene (PE) inflation film bag having the same size as mentioned above and an oxygen permeation coefficient of 2.5 x $10^{-8}$ cc•cm/(cm$^2$•sec•cmHg) were held in hot water at 130°C or in steam for 30 minutes in an autoclave, and the bags were taken out from the autoclave and allowed to stand still at a temperature of 25°C and a relative humidity of 50% for 24 hours. The tensile strength and elongation were measured according to JIS Z-1702. The obtained results are shown in Table 1.

4

Table 1

| | Example 1 PBT Film (70μm) | | Comparative Example 1 PP Film (70μm) | | Comparative Example 2 PE Film (70μm) | |
|---|---|---|---|---|---|---|
| Units | tensile strength kg/cm² | tensile elongation % | tensile strength kg/cm² | tensile elongation % | tensile strength kg/cm² | tensile elongation % |
| Untreated | 600 | 420 | 230 | 650 | 160 | 560 |
| Hot Water Treatment at 130°C for 30 minutes | 570 | 410 | 320 | 530 | melted | |
| Steam Treatment at 130°C for 30 minutes | 590 | 410 | 310 | 530 | melted | |

It is seen that the PBT bag retained the strength and elongation after the hot water treatment and the steam treatment. It also is seen that the PBT bag had a strength 2 to 2.5 times as high as the strength of the PP bag having the same thickness. Therefore, it was confirmed that a bag having the same strength can be prepared from polybutylene terephthalate with a thickness of 1/2 or less of the PP bag.

Example 2 and Comparative Examples 3 and 4

A polybutylene terephthalate (PBT) inflation film bag having a thickness of 70 $\mu$m, a length of 1 m, and width of 50 cm, an intrinsic viscosity of 1.4 as measured by using an o-chlorophenol solution at 25°C and an oxygen permeation coefficient of 4.5 x 10$^{-10}$ cc•cm/(cm$^2$•sec•cmHg), the bag used in Comparative Example 1 and a bag comprising a deodorant-containing PE bag (having a thickness of 70 $\mu$m) on the inner side of the bag of Comparative Example 1 were filled with 2 kg of absorbent cotton and cloth impregnated with body fluids, blood and excretions, which were discarded from a hospital. The opening of each bag was bound by a rubber band and each bag was heat-treated with steam at 130°C for a predetermined time. The presence or absence of a smell, the change of the shape of the bag and the sterilization state were checked by the organoleptic test. The obtained results are shown in Table 2. The sterilizationstate was good in all of the bags. However, in the bag composed of PP alone, a bad smell was emitted to the outside of the bag, and hence, the inner bag of deodorant-containing PE had to be used. In contrast, the bag of PBT had a practical utility.

## Table 2

| | | Pressure (at mospheres) | Temperature (°C) | Heating Time (minutes) | Emission of smell | Change of shape of bag |
|---|---|---|---|---|---|---|
| Example 2 | PBT bag | 1.5 | 121 | 20 | not observed | not changed |
| | | 1.5 | 121 | 30 | ditto | ditto |
| | | 1.45 | 115 | 45 | ditto | ditto |
| Comparative Example 3 | PP bag | 1.5 | 121 | 20 | observed | ditto |
| | | 1.5 | 121 | 30 | ditto | ditto |
| | | 1.45 | 115 | 45 | ditto | ditto |
| Comparative Example 4 | PP bag + deodorant-containing PE bag | 1.5 | 121 | 20 | not observed | PE bag deformed |
| | | 1.5 | 121 | 30 | not observed | ditto |
| | | 1.45 | 115 | 45 | slightly observed | ditto |

Example 3 and Comparative Example 5

A PBT inflation film bag having a thickness of 30 μm, a length of 20 cm, a width of 10 cm and an oxygen permeation coefficient of 3.0 × 10⁻¹⁰ cc·cm/(cm²·sec·cmHg) was filled with 2 g of absorbent cotton containing 0.5 g of skatole (3-methylindole) or trimethylamine absorbed thereto, and the bag was heat-sealed. The bag was placed in a desiccator and stored at a temperature of 23°C and a relative humidity of 50%. The smell was checked at intervals of 4 hours, and the time when the smell was first felt was measured.

For comparison, a PP inflation film bag having an oxygen permeation coefficient of 1.1 x 10$^{-8}$ cc•cm/- (cm$^2$•sec•cmHg) was filled with the same substance as described above, and the bag was evaluated in the same manner as described above.

The obtained results are shown in Table 3.

Table 3

| Substance | Example 3 (PBT Bag) | Comparative Example 5 (PP Bag) |
|---|---|---|
| skatole | 48 hours | 4 hours |
| trimethylamine | 48 hours | 4 hours |

Example 4 and Comparative Example 6

With respect to a PBT inflation film having a thickness of 70 $\mu$m and an oxygen permeation coefficient of 2.0 x 10$^{-10}$ cc•cm/(cm$^2$•sec•cmHg) and a polypropylene inflation film having a thickness of 70 $\mu$m and an oxygen permeation coefficiency of 1.1 x 10$^{-8}$ cc•cm/(cm$^2$•sec•cmHg), the tensile strength was measured at various predetermined temperatures. The obtained results are shown in Table 4.

As is seen from Table 4, the tensile strength of the PBT film was about 3 times as high as the tensile strength of the PP film at respective temperatures, and the temperature dependency of the tensile strength of the PBT film is smaller. Namely, it is seen that the PBT film is excellent over the PP film in the heat resistance.

Table 4

| | | | 23°C | 45°C | 55°C | 62°C | 70°C | 80°C |
|---|---|---|---|---|---|---|---|---|
| Example 4 | PBT inflation film (70 $\mu$m) | tensile strength (kg/cm$^2$) | 605 | 399 | 332 | 288 | 255 | 225 |
| Comparative Example 6 | PP inflation film (70 $\mu$m) | tensile strength (kg/cm$^2$) | 243 | 148 | 109 | 99 | 92 | 80 |

Examples 5-6, Comparative Example 7

A commercially available PP bag, colored in green, having a thickness of 60 $\mu$m, a length of 500 mm, a width of 700 mm, and a PBT bag colored in green, having a thickness of 30 $\mu$m, a length of 500 mm, a width of 700 mm, an oxygen permeation coefficient at 100 °C of 2.0 x 10$^{-10}$ cc•cm/(cm$^2$•sec•cmHg), a humidity permeation of 10 x 10$^{-3}$(g•m/m$^2$•day), and a PBT bag colored in green, having a thickness of 60 $\mu$m, a length of 500 mm, a width of 700mm, an oxygen permeation coefficient at 100°C of 2.0 x 10$^{-10}$ cc•cm/(cm$^2$•sec•cmHg), a humidity permeation of 10 x 10$^{-3}$ (g•m/m$^2$•day), were each filled with sheets of cloth, paper, cotton, gauze, and about 3 kg of syringes which are made of polypropylene. They were further charged with a biological indicator whereby to know the state of sterilization and a culture medium, and were fastened by at the end of their opening with a nylon-made binding ribbon. They were sterilized at the temperatures and for periods of time shown in Table 5. After the treating, cultivation was conducted on the culture medium at 55°C for 48 hours. An increase seen is shown as " + " and no increase seen is shown as " - ". Leaking of a bad smell was checked by a seven-member testing panel.

The obtained results are shown in Table 5.

Table. 5

| | | Sterilizing Conditions | | State of Sterlization | Leaking of smell |
|---|---|---|---|---|---|
| | | Temperature (°C) | Heating Time (minutes) | | |
| Example 5 | PBT bag 30 $\mu$m | 121 | 20 | − | no |
| | ditto | ditto | 30 | − | ditto |
| | ditto | ditto | 40 | − | ditto |
| Example 6 | PBT bag 60 $\mu$m | ditto | 20 | − | ditto |
| | ditto | ditto | 30 | − | ditto |
| | ditto | ditto | 40 | − | ditto |
| Comp.Ex.7 | PP bag 60 $\mu$m | ditto | 20 | + | 7 felt |
| | ditto | ditto | 30 | − | 6 felt |
| | ditto | ditto | 40 | − | 6 felt |

**Claims**

1. A polybutylene terephthalate bag formed of a film of polybutylene terephthalate resin having a thickness of 10 to 200$\mu$m and an oxygen permeation coefficient of 5.0 x 10$^{-10}$ cc.cm/(cm$^2$.sec.cmHg) or below at 100$^\circ$C.

2. A bag as set forth in claim 1, in which the polybutylene terephthalate film has a thickness of 20 to 100 $\mu$m.

3. A bag as set forth in claim 1 or claim 2, in which the polybutylene terephthalate film has an oxygen permeation coefficient of 3.0 x 10$^{-10}$ cc.cm/(cm$^2$.sec.cmHg) or below at 100$^\circ$C.

4. A bag as set forth in any preceding claim, in which the film is a cylindrical film formed by the inflation method from a polybutylene terephthalate resin having an intrinsic viscosity of at least 1.0 and the bag is formed therefrom by cutting the cylindrical film to a predetermined length and heat-sealing one end thereof.

5. A bag as set forth in claim 4, in which the film is formed from a polybutylene terephthalate resin having an intrinsic viscosity in the range 1.0 to 2.5.

6. A bag as set forth in claim 4 or claim 5, in which the film is an opaque film formed by the inflation method without quenching.

7. A bag as set forth in any one of claims 1 to 3, in which the film is formed by the T-die method from a polybutylene terephthalate resin having an intrinsic viscosity of at least 0.5.

8. A bag as set forth in any preceding claim, in which the polybutylene terephthalate film has a humidity permeation of 7 to 10 x 10$^{-3}$ g.m/m$^2$.day.

9. A bag retaining unpleasant odours, formed by placing odoriferous waste in a bag as set forth in any preceding claim, closing the bag and treating the closed bag with hot air, steam or hot water.

10. A sterilized bag for waste material, as set forth in any preceding claim.

**Patentansprüche**

1. Polybutylenterephthalat-Beutel, gebildet aus einer Folie aus Polybutylenterephthalat-Harz mit einer Dicke von 10 bis 200 $\mu$m und einem Durchlässigkeits-Koeffizienten für Sauerstoff bei 100 $^\circ$C von 5,0 x 10$^{-10}$ cm$^3$•cm/(cm$^2$•s•cm Hg) oder weniger hat.

2. Beutel nach Anspruch 1, worin die Polybutylenterephthalat-Folie eine Dicke von 20 bis 100 $\mu$m hat.

3. Beutel nach Anspruch 1 oder Anspruch 2, worin die Polybutylenterephthalat-Folie einen Durchlässigkeits-Koeffizienten für Sauerstoff bei 100 $^\circ$C von 3,0 x 10$^{-10}$ cm$^3$•cm/(cm$^2$•s•cm Hg) oder weniger hat.

4. Beutel nach irgendeinem vorhergehenden Anspruch, worin die Folie eine zylindrische Folie ist, die mittels des Aufblas-Verfahrens aus einem Polybutylenterephthalat-Harz mit einer Grenzviskosität von wenigstens 1,0 gebildet ist und der Beutel daraus durch Zerschneiden der zylindrischen Folie auf eine vorher festgelegte Länge und Heißversiegeln des einen Endes derselben gebildet wird.

5. Beutel nach Anspruch 4, worin die Folie aus einem Polybutylenterephthalat-Harz mit einer Grenzviskosität im Bereich von 1,0 bis 2,5 gebildet ist.

6. Beutel nach Anspruch 4 oder Anspruch 5, worin die Folie eine undurchsichtige Folie ist, die mittels des Aufblas-Verfahrens ohne Abschrecken gebildet ist.

**7.** Beutel nach irgendeinem der Ansprüche 1 bis 3, worin die Folie mittels des T-Düsen-Verfahrens aus einem Polybutylenterephthalat-Harz mit einer Grenzviskosität von wenigstens 0,5 gebildet ist.

**8.** Beutel nach irgendeinem vorhergehenden Anspruch, worin die Polybutylenterephthalat-Folie eine Feuchtigkeits-Durchlässigkeit von 7 bis 10 x $10^{-3}$ g•m/m$^2$•d hat.

**9.** Beutel, der unangenehme Gerüche, die durch übelriechende Abfälle entstehen, zurückhält, durch Einbringen der übelriechenden Abfälle in einen Beutel nach irgendeinem vorhergehenden Anspruch, Verschließen des Beutels und Behandeln des verschlossenen Beutels mit Heißluft, Wasserdampf oder heißem Wasser.

**10.** Sterilisierter Beutel für Abfallmaterialien nach irgendeinem vorhergehenden Anspruch.

**Revendications**

**1.** Sac en polytéréphtalate de butylène, formé d'une pellicule en résine de polytéréphtalate de butylène ayant une épaisseur de 10 à 200 $\mu$m et un coefficient de perméation de l'oxygène de 5,0x$10^{-10}$ cm$^3$.cm/(cm$^2$.sec.cm de Hg) ou moins à 100 °C.

**2.** Sac selon la revendication 1, dans lequel la pellicule de polytéréphtalate de butylène a une épaisseur de 20 à 100 $\mu$m.

**3.** Sac selon la revendication 1 ou la revendication 2, dans lequel la pellicule de polytéréphtalate de butylène a un coefficient de perméation de l'oxygène de 3,0x$10^{-10}$ cm$^3$.cm/(cm$^2$.sec.cm de Hg) ou moins à 100 °C.

**4.** Sac selon l'une quelconque des revendications précédentes, dans lequel la pellicule est une pellicule cylindrique formée par le procédé de soufflage à partir d'une résine de polytéréphtalate de butylène ayant une viscosité intrinsèque d'au moins 1,0, et le sac est formé à partir de celle-ci en découpant la pellicule cylindrique à une longueur prédéterminée et en thermosoudant une extrémité de celle-ci.

**5.** Sac selon la revendication 4, dans lequel la pellicule est formée à partir d'une résine de polytéréphtalate de butylène ayant une viscosité intrinsèque dans la gamme de 1,0 à 2,5.

**6.** Sac selon la revendication 4 ou la revendication 5, dans lequel la pellicule est une pellicule opaque formée par le procédé de soufflage sans refroidissement brusque.

**7.** Sac selon l'une quelconque des revendications 1 à 3, dans lequel la pellicule est formée par le procédé à la filière en T à partir d'une résine de polytéréphtalate de butylène ayant une viscosité intrinsèque d'au moins 0,5.

**8.** Sac selon l'une quelconque des revendications précédentes, dans lequel la pellicule de polybutylène téréphtalate a une perméation de l'humidité de 7 à 10x$10^{-3}$ g.m/m$^2$.jour.

**9.** Sac retenant les odeurs désagréables, formé en plaçant des déchets odoriférants dans un sac selon l'une quelconque des revendications précédentes, en fermant le sac et en traitant le sac fermé à l'air chaud, la vapeur ou l'eau chaude.

**10.** Sac stérilisé pour déchets, selon l'une quelconque des revendications précédentes.